# EUROPEAN PATENT SPECIFICATION

(11) **EP 4 487 794 B1**
(45) Date of publication and mention of the grant of the patent: **22.04.2026**
(21) Application number: 24186712.6
(22) Date of filing: 05.07.2024
(51) Int. Cl.: A61B 17/17, A61B 17/80

(54) **MODULAR AIMING BLOCK FOR TARGETING SYSTEM FOR BONE SURGERY**
MODULARER ZIELBLOCK FÜR ZIELSYSTEM FÜR KNOCHENCHIRURGIE
BLOC DE VISÉE MODULAIRE POUR SYSTÈME DE VISÉE POUR LA CHIRURGIE OSSEUSE

(30) Priority: 06.07.2023 US 202363525231 P
(43) Date of publication of application: 08.01.2025
(73) Proprietor: Stryker European Operations Limited, Carrigtwohill, Co. Cork T45 HX08 (IE)
(72) Inventor: FRÖHLICHER, Christian, 4514 Solothurn (CH); BOUQUET, Joël, 4514 Lommiswil (CH)
(74) Representative: Schott, Jakob Valentin

(56) References cited:
- EP-A1- 3 375 392
- US-A1- 2018 200 065
- US-B2- 11 065 013
- US-B2- 11 432 832

## Description

### BACKGROUND

The present disclosure relates to aiming systems used in conjunction with bone plates and methods of use thereof for the fixation of fractures of the bone, such as the femur, tibia, humerus, and radius. More specifically, the present disclosure includes a bone plate targeting system including a targeting block/aiming block, an aiming arm, and a handle for connecting the targeting block and aiming arm to a bone plate.

When a bone is fractured or otherwise damaged, bone plates are oftentimes attached to an outer surface of the damaged bone to provide stabilization to the bone and promote healing. These bone plates typically have a bone-contacting side and an opposing side facing away from the bone. A plurality of openings extend entirely through the two surfaces. Such openings may be designed for polyaxial and/or monoaxial screw placement and may accommodate other fixation features such as sutures.

In order to accurately place the screws within the plurality of openings, especially in less invasive procedures, targeting guides are often used to ensure a proper alignment. Such targeting guides may be suspended above the surface of the bone plate and various sleeves can be positioned through a plurality of holes in the targeting guide to align with the plurality of openings in the bone plate. Such targeting guides are not without drawbacks. Traditional targeting guides require multiple points of connection between the guide and the bone plate. Further, to provide coverage for the entire bone plate, traditional targeting guides are often bulky and obstruct the view of operators. Accordingly, an improvement in targeting guides that provides coverage for entire bone plates in a minimally invasive manner is desired.

EP 3 375 392 A1 discusses bone plates for engaging bone members. The bone plates can receive one or more screws to secure the bone plates to an underlying bone member. The one or more screws can be inserted into bone plate holes that can be considered locking or non-locking. The bone plates described can have particular combinations of locking and/or non-locking holes. In addition, instruments such as distal and proximal aiming guides can accompany the bone plates to guide one or more screws into the bone plates.

### BRIEF SUMMARY

A targeting guide is discussed in accordance with independent claim 1, to which the reader should now refer. Specific embodiments are discussed in the dependent claims. According to one example of the present disclosure, a targeting guide for aligning a bone plate comprises an aiming arm; an attachment portion extending from a proximal end of the aiming arm, the attachment portion having an opening removably connectable with a shaft of a handle; and an aiming block/targeting block removably connectable with the shaft.

In another aspect, the aiming arm extends along a longitudinal axis and has an upper side and an opposing lower side, a first sidewall and a second sidewall connecting the upper side to the lower side, and a plurality of holes extending through the upper and lower sides.

In a different aspect, the targeting block has an upper surface, an opposing lower surface, a sidewall connecting the upper surface to the lower surface, and a plurality of apertures extending through the upper and lower surfaces.

In yet another aspect, the targeting guide includes an outrigger extending laterally away from the first lateral side of the aiming arm.

In another aspect, the outrigger extends away from the first lateral side and the second lateral side of the aiming arm.

In a different aspect, the outrigger includes a hole defining an axis, the axis extending toward a neck portion of the bone plate.

In a further aspect, the outrigger is integrally attached to the aiming arm.

In another aspect, the outrigger is attached to the aiming arm with a fastener.

In yet a further aspect, the guide includes a locking collar attachable with the shaft of the handle.

In a different aspect, the sidewall of the targeting block includes five sidewalls defining a substantially pentagonal shape.

In another aspect, the sidewall of the targeting block is curved to define a circular targeting block.

In a different aspect, each hole of the plurality of holes of the aiming arm aligns with a corresponding plate hole of a shaft portion of the bone plate.

In a further aspect, each aperture of the plurality of apertures of the targeting block aligns with a corresponding plate hole of a head portion of the bone plate.

In another aspect, the handle is configured to connect the aiming arm to at least one of a head portion and neck portion of the bone plate.

In a different aspect, the attachment portion is integrally attached to the aiming arm.

In another aspect, the shaft of the handle is configured to extend through the opening in the attachment portion and a block opening in the targeting block.

In yet another aspect, the targeting block includes a groove for receiving a portion of the attachment portion and the aiming arm.

According to another aspect of the present disclosure, a targeting guide system for aligning a bone plate comprises an aiming arm having an attachment portion extending from a proximal end thereof and a plurality of arm holes extending through an upper and lower side of the aiming arm; a targeting block defining a connection hole and a plurality of block holes extending through an upper and lower surface of the targeting block; a bone plate including a head portion and a shaft portion, each of the head and shaft portions having a plurality of holes extending therethrough that substantially align with corresponding holes of the plurality of arm holes and block holes; and a handle removably connectable with a handle hole in the bone plate, an opening in the attachment portion of the aiming arm, and the connection hole in the targeting block.

In another aspect, the attachment portion is integral with and extends from the proximal end of the aiming arm.

In a further aspect, the targeting block includes a groove defined by a bottom surface of the targeting block, the groove configured to receive at least a portion of the attachment portion.

In another aspect, in an attached configuration, the targeting block is removably connected to the handle such that a top surface of the targeting block is flush with a top side of the aiming arm.

In a different aspect, the handle further comprises a shaft extending from a gripping portion.

In another aspect, the gripping portion is configured to rotationally align the aiming arm with the bone plate.

In a further aspect, the shaft is configured to extend through the opening in the attachment portion of the aiming arm and the connection hole in the targeting block.

In another aspect, the handle further comprises a leg extending laterally away from a longitudinal axis of the handle.

According to another aspect of the present disclosure, a method of aligning a bone plate, the method comprises attaching a handle to an aperture in a bone plate; inserting a shaft of the handle through a hole in an attachment portion of an aiming arm; inserting the shaft of the handle through a connection hole in a targeting block.

In another aspect, the method further comprises moving the targeting block along the shaft such that a groove defined by a lower surface of the targeting block receives at least a portion of the attachment portion of the aiming arm.

In yet another aspect, the moving step includes moving the targeting block along the shaft such that a groove within the connection hole receives a corresponding ring of the shaft to secure the targeting block to the shaft.

In a further aspect, the method further comprises threading a locking collar to the shaft.

In another aspect, the method further comprises inserting a drill sleeve through an outrigger extending from a lateral surface of the aiming arm.

In a different aspect, the inserting the drill sleeve step further includes inserting a distal end of the drill sleeve into an aperture in a neck portion of the bone plate.

In another aspect, the attaching step includes attaching the handle to an aperture in a head portion of the bone plate.

In a further aspect, the attaching step includes attaching the handle to an aperture in a neck portion of the bone plate.

In a different aspect, the method further comprises aligning a plurality of arm holes extending through the aiming arm with a corresponding plurality of apertures in a shaft portion of the bone plate.

In yet another aspect, the moving step includes moving the targeting block along the shaft such that the targeting block is level with the aiming arm.

In a different aspect, the attaching step includes attaching a distal end of the handle in an aperture in a head portion of the bone plate and a leg of the handle in an adjacent aperture in the head portion of the bone plate.

In another aspect, the method further comprises inserting a tissue protection sleeve through a block hole extending through the targeting block.

In a different aspect, the inserting a tissue protection sleeve step includes inserting the tissue protection sleeve through the block hole such that the tissue protection sleeve is secured in place by a cantilever pin.

According to another aspect of the disclosure, a targeting guide system for aligning a bone plate includes an aiming arm connected to an attachment portion, a targeting block configured to be received on a top side of the attachment portion, and a bone plate including a head portion and a shaft portion. The aiming arm has a plurality of holes, and the targeting block has a plurality of apertures. The head portion has a plurality of holes that each align with one of the plurality of apertures in the targeting block and the shaft portion has a plurality of holes that each align with one of the plurality of holes in the aiming arm.

### BRIEF DESCRIPTION OF THE DRAWINGS

A more complete appreciation of the subject matter of the present disclosure and the various advantages thereof can be realized by reference to the following detailed description, in which reference is made to the following accompanying drawings:
FIG. 1 is a perspective, partially exploded view of an aiming system according to one embodiment of the present disclosure.
FIG. 2 is another perspective, partially exploded view of the aiming system of FIG. 1.
FIG. 3 is a top view of the aiming arm of FIG. 1.
FIG. 4 is a side view of the aiming arm of FIG. 1.
FIG. 5 is a side cross section view of the aiming arm of FIG. 1.
FIG. 6 is an enlarged perspective exploded view of a portion of the aiming system of FIG. 1.

### DETAILED DESCRIPTION

Reference will now be made in detail to the various embodiments of the present disclosure illustrated in the accompanying drawings. It should be noted that the drawings are in simplified form and are not drawn to precise scale. Additionally, the term "a," as used in the specification, means "at least one." The terminology includes the words above specifically mentioned, derivatives thereof, and words of similar import. In addition to the embodiment described herein, other variations may include aspects described herein combined in any suitable manner having combinations of all or some of the aspects described.

In describing preferred embodiments of the disclosure, reference will be made to directional nomenclature used in describing the human body. It is noted that this nomenclature is used only for convenience and that it is not intended to be limiting with respect to the scope of the present disclosure. As used herein, when referring to bones or other parts of the body, the term "anterior" means toward the front part of the body or the face, and the term "posterior" means toward the back of the body. The term "medial" means toward the midline of the body, and the term "lateral" means away from the midline of the body. The term "superior" means closer to the head, and the term "inferior" means more distant from the head. When referring to the directional nomenclature of an operator implementing the aiming system described herein, the term "proximal" means closer to the operator and the term "distal" means further from the operator.

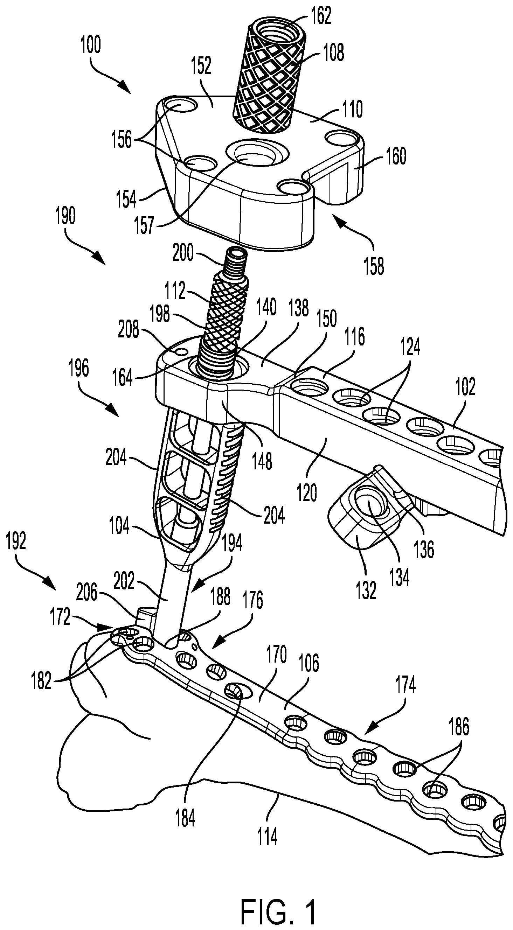

FIG. 1 illustrates an aiming system 100 according to one embodiment of the present disclosure. Aiming system 100 includes an aiming arm 102 attachable to a handle 104. A locking collar 108 secures an aiming block, or targeting block 110, to a shaft 112 of handle 104 and to aiming arm 102. Handle 104 is secured to a bone plate 106, which is secured to a bone such as the femur 114. Although the embodiments described herein and illustrated in the figures depict an aiming system 100 used in conjunction with a femoral bone plate, it is appreciated that aiming system 100 may be implemented with other bone plates and bones, such as the tibia, humerus, and the like.

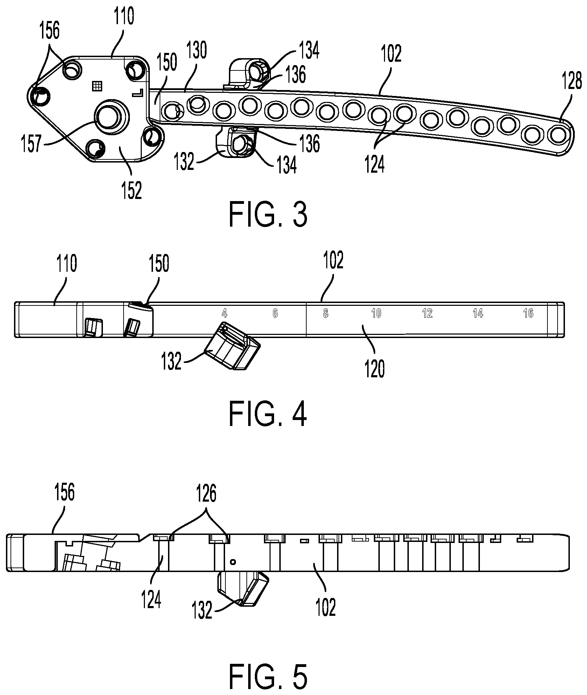

FIGS. 3-5 illustrate various views of aiming arm 102. Aiming arm 102 is in the form of a rectangular block and extends along a longitudinal axis that is substantially parallel with the longitudinal axis of femur 114. Aiming arm 102 includes an upper side 116 opposite a lower side 118. A first sidewall 120 is opposed to a second sidewall 122, each of the first and second sidewalls 120, 122 connecting the upper side 116 and lower side 118. As such, the upper side 116 is parallel to the lower side 118, and the first sidewall 120 is parallel to the second sidewall 122 to form a rectangular prism.

Aiming arm 102 includes a plurality of holes 124 extending through the upper and lower sides 116, 118 and along at least a portion of the length of aiming arm 102. Each hole 124 has a central hole axis that aligns with an aperture axis of an aperture 186 of a shaft portion 174 of bone plate 106. Thus, aiming arm 102 may be selected in accordance with a particular bone plate, such as femoral bone plate 106, such that each hole 124 is spaced apart from each other in accordance with the spacing of apertures 186 of bone plate 106. Each hole 124 may be circular, elliptical, or the like, and may be smooth, threaded, or have other features to engage various sleeves and drill bits extending therethrough. Each hole 124 may further include a ledge 126 for aiding in the securement of various drill sleeves. Certain drill sleeves include winglets that align with ledge 126 and ensure the drill sleeves are properly positioned relative to aiming arm 102.

Aiming arm 102 extends from a distal end 128 to a proximal end 130. Distal end 128 is rounded around the most distal hole 124. In alternative embodiments, distal end 128 may be blunt or may include additional holes for a second handle or connection sleeve to secure distal end 128 to a distal end of bone plate 106.

Although aiming arm 102 is described herein as being a rectangular prism having four longitudinal sides, other configurations of aiming arms are appreciated. For example, a triangular aiming arm having three longitudinal sides may be implemented. Additionally, for rectangular aiming arms, each opposing pair of sides may have different hole patterns formed therein to account for different bone plates. Such configurations are described in U.S. Pat. No. 11,432,832.

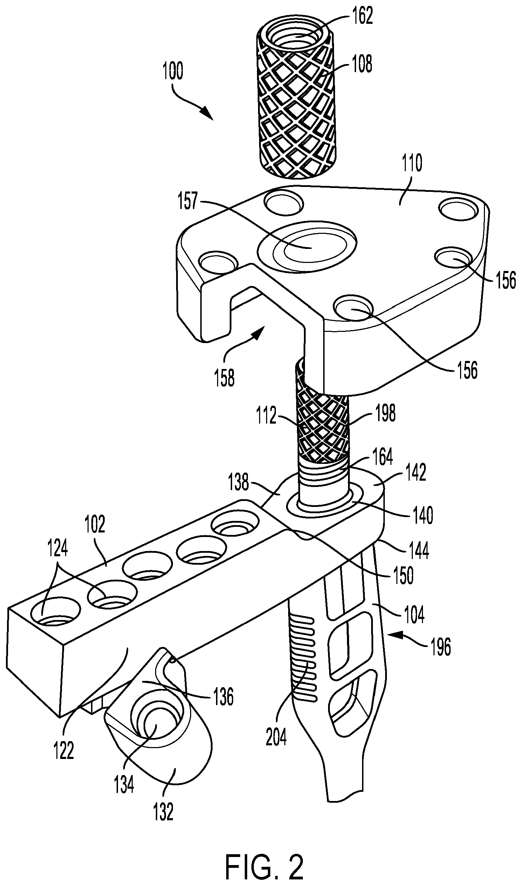

An outrigger 132 is illustrated in FIGS. 1-4 and extends laterally away from the first and second sidewalls 120, 122 of aiming arm 102 and is configured to provide alignment for sleeves and drill bits at oblique angles relative to the angles provided by holes 124 of aiming arm 102. Outrigger 132 includes two cylindrical portions 134 that provide alignment for drill sleeves and drill bits extending therethrough. Cylindrical portions 134 are preferably recessed beneath the upper side 116 of aiming arm 102 to avoid snagging on tissue adjacent aiming arm 102 in use. Outrigger 132 is also positioned beneath the upper side 116 of aiming arm 102 in order for various fixed length tissue protection sleeves to extend through cylindrical portions 134 toward bone plate 106. Outrigger 132 is preferably integrally formed with aiming arm 102 at a position that may correspond to holes in the neck portion 176, head portion 172, or shaft portion 174 of bone plate 106. As illustrated, outrigger 132 is positioned approximately ¼ the distance across the length of aiming arm 102 as measured from the proximal end 130. Aiming arm 102 may be manufactured in ways that outrigger 132 is integrally attached to aiming arm 102 and that each cylindrical portion 134 defines a cylinder axis that is optimized at an oblique angle relative to the hole axes of aiming arm 102. This oblique angle allows an operator to access holes in the neck portion 176 of bone plate 106, which would otherwise be difficult to access in existing aiming systems for minimally invasive procedures. Further, each cylindrical portion 134 preferably extends laterally from the aiming arm 102 such that only a narrow bridge 136 of material separates cylindrical portion 134 from aiming arm 102, thus reducing the overall width of aiming arm 102 to a width that is smaller tha targeting block 110.

In alternative embodiments, outrigger 132 may extend from only one lateral sidewall of aiming arm 102 and include only a single cylindrical portion. Furthermore, outrigger 132 may sit flush with the upper side 116 of aiming arm 102 or may be raised above the upper side 116. While outrigger 132 is illustrated as being integrally attached with aiming arm 102, it is foreseen that outrigger 132 may be manufactured as a separate and distinct component that is attached to aiming arm 102 to provide adjustability to the cylindrical portion of the outrigger. As such, the outrigger may attach to the aiming arm with or without the use of fasteners, and may have distinct grooves or other attachment features that allow an operator to lock the outrigger into the aiming arm such that the cylindrical portion aims at a particular hole of the underlying bone plate. In some applications, an aiming arm 102 may be utilized without an outrigger depending on the location and type of bone fracture and whether there is enough space to utilize an outrigger.

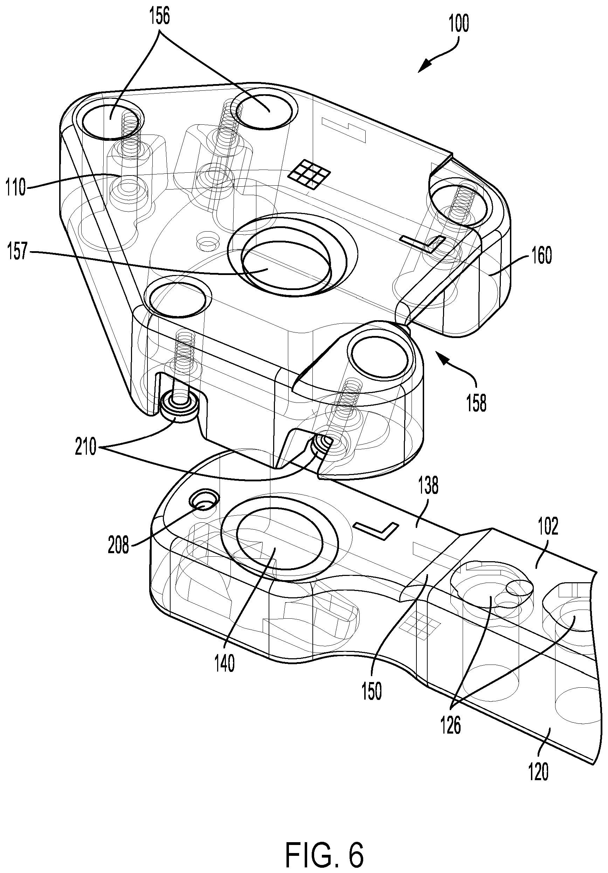

FIGS. 1-2 and 6 illustrate an attachment portion 138 extending proximally from the proximal end 130 of aiming arm 102. Attachment portion 138 provides a secure connection point between aiming arm 102 and handle 104. As such, attachment portion 138 is preferably integrally connected with aiming arm 102 and extends proximally at a distance corresponding to a particular targeting block 110 that may be utilized with that particular aiming arm 102. Attachment portion 138 includes an attachment hole 140 configured to receive a shaft 112 of handle 104. Attachment hole 140 is preferably smooth such that an operator can slide aiming arm 102 over shaft 112 without needing to rotate aiming arm 102 to accommodate threads or other attachment features along shaft 112. Attachment hole 140 extends from a top side 142 through a bottom side 144 of attachment portion 138. A sidewall 146 connects the top and bottom sides 142, 144 and may have various curved regions 148 to minimize the footprint of attachment portion 138 for minimally invasive procedures. A ramp 150 connects the proximal end 130 of aiming arm 102 with attachment portion 138 and lowers the top side 142 of attachment portion 138 slightly beneath the upper side 116 of aiming arm 102. Ramp 150 allows a targeting block 110 to sit above attachment portion 138 and flush with aiming arm 102 without extending above aiming arm 102. As such, ramp 150 may be dimensioned according to the particular targeting block implemented in each application. Attachment portion 138 further includes a leg hole 208 for receiving a leg (not shown) extending proximally from gripping portion 204 of handle 104 hole for receiving a portion of either handle 104 and/or targeting block 110.

FIGS. 1-2 and 6 illustrate a targeting block 110 according to one embodiment of the present disclosure. Targeting block 110 includes an upper surface 152, a lower surface 154, and a sidewall defined between and connecting the upper and lower surfaces 152, 154. A plurality of block holes 156 extend through the upper and lower surfaces 152, 154 of targeting block 110. Each block hole 156 defines a block hole axis that substantially aligns with an aperture 182 in the head portion 172 of bone plate 106. Each block hole 156 may further include a distally extending cantilever pin 210 positioned within or adjacent block hole 156. Each cantilever pin 210 may bias toward the central block hole axis and move laterally away from the central block hole axis when a force is applied to the cantilever pin 210. Accordingly, when a tissue protection sleeve is inserted through block hole 156, the tissue protection sleeve may push the cantilever pin 210 away from the central block hole axis such that the cantilever pin 210 holds the tissue protection sleeve in place via friction. Targeting block 110 is preferably a pentagram shape to maintain a small profile while securely attaching to aiming arm 102. In alternative embodiments, targeting block 110 may define circular, rectangular, or another shape. Targeting block 110 includes a block attachment hole 157 configured to receive shaft 112 of handle 104. Block attachment hole 157 may be substantially smooth such that targeting block 110 need not be rotated to translate along shaft 112. Various knurling or other locking features may be disposed along the length of shaft 112 to secure the targeting block 110 to shaft 112 without the need for rotation.

Targeting block 110 includes a groove 158 defined in the lower surface 154 that is configured to receive at least a portion of attachment portion 138. Groove 158 extends proximally from a distal surface 160 of targeting block 110 at least partially across a length of targeting block 110. As such, groove 158 may be in operative communication with block attachment hole 156. Groove 158 is dimensioned to receive at least a portion of attachment portion 138 and/or aiming arm 102 to prevent targeting block 110 from rotating relative to aiming arm 102. Groove 158 further allows upper surface 152 of targeting block 110 to sit flush with the upper side 116 of aiming arm 102. Groove 158 may include a ramped portion (not shown) that corresponds to the ramp 150 of attachment portion 138. The ramped portion of the groove 158 may further facilitate placement of the targeting block 110 over the attachment portion 138 of aiming arm 102.

FIGS. 1-2 depict a locking collar 108. Locking collar 108 is substantially cylindrical and includes an outer diameter greater than the diameter of block hole 156 to prevent locking collar 108 from passing through block hole 156. Locking collar 108 includes a cannula 162 that is at least partially threaded to secure cannula 162 to corresponding threads 164 of shaft 112. An outer surface 166 of locking collar 108 may be knurled or include other textures or features to aid a user in grasping and rotating locking collar 108 over shaft 112. In alternative embodiments, cannula 162 may be unthreaded and may be secured to shaft 112 by securing to various ribs or other protrusions on shaft 112.

FIG. 1 depicts a bone plate 106. Bone plate 106 is described and illustrated herein as a femoral bone plate, although it is contemplated that bone plate 106 may be other implanted with other bone plates. Bone plate 106 is elongated and includes a bone-contacting surface and an outward facing surface 170. Bone plate 106 includes a head portion 172 at the proximal end, a shaft portion 174 at a distal end, and a neck portion 176 connecting the head and shaft portions 172, 174. Each of the head, neck, and shaft portions has a plurality of respective apertures 182, 184, 186 extending through the bone-contacting and outward facing surfaces. The plurality of apertures 182, 184, 186 are configured for polyaxial or monoaxial placement of screws within the apertures. Plates with such apertures are described in U.S. Pat. No. 11,039,865. Additionally, bone plate 106 includes a connection aperture 188 positioned in the head portion 172 configured to receive a portion of handle 104. The connection aperture 188 may include locking features such as threads, grooves, or the like to engage a portion of handle 104. Although connection aperture 188 is illustrated as being formed in the head portion 172 of bone plate 106, it is foreseeable that connection aperture 188 is formed through the neck portion 176 or shaft portion 174 of bone plate 106.

FIGS. 1-2 illustrate a handle 104. Handle 104 extends along a longitudinal axis and includes a shaft 112 at the proximal end 190, a connecting portion 194 at the distal end 192, and an intermediary portion 196 therebetween. Shaft 112 is configured to secure the targeting block 110 to the attachment portion 138 of aiming arm 102 with a locking collar 108. As such, shaft may have studs 198, threads 164, ribs, or other attachment features that can either rotatably or non-rotatably secure at least one of aiming arm 102 and targeting block 110 to shaft 112. A decreased-diameter shaft 200 extends proximally from shaft 112 and may be color-coded to help visualize when decreased-diameter shaft 200 extends through locking collar 108. Intermediary portion 196 tapers outward from shaft 112 to define two diametrically opposed gripping portions 204 with a central shaft 112 therebetween. Gripping portions 204 include a texture that allows an operator to easily maneuver the handle 104 in place to connect with bone plate 106. Intermediary portion 196 tapers at the distal end into connection shaft 202. Connection shaft 202 may be substantially smooth and may include an attachment feature, such as a threaded or tapered distal tip (not shown) to facilitate a connection and detachment with the connection aperture 188 of bone plate 106. Connection shaft 202 may further include a leg 206 extending laterally away from connection shaft to engage a second connection aperture of bone plate 106. Such a leg configuration prevents handle 104 from rotating relative to bone plate 106.

A method of using the aiming system 100 is described herein. An operator may first surgically prepare the tissue and bone for implementation of aiming system 100. One advantage of aiming system 100 over conventional aiming systems if the low profile of aiming arm 102 and targeting block 110, which result in greater visibility for operators and smaller incisions required for the patient. Further, aiming system 100 is advantageous as it only requires a single rigid connection between the aiming arm 102 and bone plate 106 via handle 104, which also increases the space underneath aiming arm 102 in which an operator can maneuver various drill sleeves. Depending on the particular type and location of fracture, an operator may further choose to implement only one of the aiming arm 102 and targeting block 110. For example, if drill sleeves are only required for a shaft portion 174 of bone plate 106, an operator may attach aiming arm 102 to handle 104, but not attach targeting block 110 to handle 104. Such an arrangement allows for increased visibility in the space surrounding aiming arm 102 while maintaining a smaller profile than an aiming system with aiming arm 102 and targeting block 110.

Moving to the implementation of aiming system 100. An advantage of aiming system 100 is that it allows an operator the choice of securing the aiming arm 102, targeting block 110, and/or bone plate 106 together either outside of the surgical area or inside the surgical area. If the operator desires to attach the components of aiming system 100 together outside of the surgical area, the operator may first attach handle 104 to bone plate 106. Such an attachment is facilitated by placing the connection shaft 202 of handle 104 into a corresponding connection aperture 188 in bone plate 106. As described herein, connection aperture 188 may be positioned in the head portion 172, neck portion 176, or shaft portion 174 of bone plate 106. An attachment feature such as a tapered distal tip or threaded distal tip of connection shaft 202 may facilitate the connection between connection shaft 202 and connection aperture 188, and such a connection may result in a tactile click or snap to indicate to an operator that the handle 104 is secured to bone plate 106. Further, to prevent rotation of handle 104 relative to bone plate 106, an operator may secure leg 206 to a leg connection hole (not shown) of bone plate 106. Such a leg connection hole may be in the head portion 172, neck portion 176, or shaft portion 174 of bone plate 106.

An operator may then position bone plate 106 against femur 114 and apply pressure to bone plate 106 to hold it in place while the remaining components of aiming system 100 are attached to handle 104. In embodiments where outrigger 132 is a separate component from aiming arm 102, outrigger 132 may be attached to aiming arm 102 in a manner that allows the cylindrical portions 134 of outrigger 132 to define a central axis that extends towards an aperture 184 in the neck portion 176 of bone plate 106. Alternatively, if the outrigger 132 is integral with aiming arm 102, aiming arm may be directly attached to handle 104 without additional steps. An operator may generally align aiming arm 102 such that a longitudinal axis of aiming arm 102 is parallel to the longitudinal axis of bone plate 106. Attachment portion 138 may then be aligned with shaft 112 of handle 104 such that shaft 112 is concentric with attachment hole 140. Aiming arm 102 is moved distally until shaft 112 extends through attachment hole 140 of attachment portion 138. Aiming arm 102 may be fully secured in place when a tactile response, such as a click or snap is caused by a ring of shaft 112 extending into a corresponding groove of attachment hole 140. Further, to prevent rotation of aiming arm 102 relative to handle 104 and to ensure alignment between holes 124 of aiming arm and apertures 186 in shaft portion of bone plate 106, an upper leg (not shown) extending proximally from the gripping portion 204 of handle 104 may extend into a leg hole 208 of attachment portion 138. In embodiments where outrigger 132 is adjustable relative to aiming arm 102, an operator may then adjust outrigger 132 to a desired position.

Next, an operator may attach targeting block 110 to shaft 112. An operator may first align targeting shaft 112 with block attachment hole 157 such that shaft 112 is concentric with block attachment hole 157. Additionally, groove 158 is aligned with aiming arm 102. Targeting block 110 is then advanced distally until a lower surface of targeting block 110 contacts attachment portion 138 and groove 158 extends at least partially over ramp 150 and upper side 116 of aiming arm 102 to sit flush with the upper side 116 of aiming arm 102. To alert to an operator that targeting block 110 is positioned correctly over attachment portion 138, a tactile response such as a click or snap may be facilitated by various attachment features between block attachment hole 157 and shaft 112. Locking collar 108 may then be advanced over shaft 112 such that internal threading of cannula 162 engages corresponding external threading of shaft portion 174.

To remove targeting block 110 from shaft 112, locking collar 108 is first removed from shaft 112 by rotating locking collar 108 in the opposite direction as it was rotated during attachment. Once removed, an operator may pull targeting block 110 proximally off shaft 112. Likewise, to remove aiming arm 102 from shaft 112 of handle, aiming arm 102 may be pulled proximally to release attachment portion 138 from shaft portion 174. To remove handle 104 from bone plate 106, in operator may pull handle 104 proximal while simultaneously maneuvering handle 104 such that the distal end of connection shaft 202 pulls away from connection aperture 188. Such a removal process simplifies often complicates removes processes and allows for minor and major corrections to be achieved during surgery in a minimally invasive manner.

In an alternative embodiment in which aiming arm 102 is not implemented with aiming system 100, an operator may first attach handle 104 to bone plate 106 using the same methods described herein, and then may place bone plate 106 against femur 114. Then, instead of attaching aiming arm 102 to shaft 112 before attaching targeting block 110, targeting block 110 is aligned directly over shaft 112 such that shaft 112 is concentric with block hole 156. Targeting block 110 is then lowered over shaft 112 and may elicit a tactile response such as a click or snap when secured in place above the gripping portions 204. To ensure rotational alignment of targeting block 110 relative to handle 104 and bone plate 106, groove 158 of targeting block 110 may be positioned above the gripping portion 204 extending on the distal side of the handle 104. A locking collar 108 may be threaded over shaft 112 to prevent targeting block 110 from moving proximally over shaft 112. Alternatively, the lower surface of targeting block 110 may include a protrusion that extends within a leg hole 208 of attachment portion 138. In this embodiment, an operator can drill through targeting block 110 into the head portion 172 of bone plate 106 without needing to cause a more invasive procedure in which an aiming arm 102 is additionally implemented.

In yet another alternative embodiment in which only an aiming arm 102 is utilized with aiming system 100 and not a targeting block 110, an operator may first attach handle 104 to bone plate 106 using the same methods described herein, and then may place bone plate 106 against femur 114. Once bone plate 106 and handle 104 are properly positioned, an operator may concentrically align shaft 112 of handle 104 with the attachment hole 140 of attachment portion 138. Aiming arm 102 may then be lowered over shaft 112 until aiming arm 102 contacts the gripping portion 204 of handle 104 and/or elicits a tactile response to an operator that the aiming arm 102 is properly positioned. To ensure rotational alignment of aiming arm 102 relative to handle 104 and bone plate 106, an upper leg (not shown) of handle 104 may be positioned within the leg hole 208 of attachment portion 138. Locking collar 108 may then be inserted over shaft 112 to prevent aiming arm 102 from translating proximally in use.

In use, once each component of aiming system 100 is in place, drill sleeves may be inserted through each of aiming arm holes 124, cylindrical portion 134 of outrigger 132, and targeting block holes 156 and into respective apertures of bone plate 106. Such drill sleeves ensure a proper alignment of drill bits and fasteners inserted therethrough while preventing damage to the surrounding tissue. Drill sleeves may be secured in place via ledges 126 formed within various holes of the aiming arm or by other securement means.

Each component described herein for aiming system 100 may be provided in a kit. As such, a particular femoral fracture fixation kit may include a femoral bone plate 106, a handle 104, an aiming arm 102, a targeting block 110, and a locking collar 108. An operator may then select each component according to the particular fracture application. For example, if a fracture only requires extensive drilling through a shaft portion 174 of bone plate 106, an operator may not use a targeting block 110 and may only use aiming arm 102, locking collar 108, and handle 104. Thus, an aiming system kit allows an operator to have the option of selecting any component necessary to fix a particular fracture.

Components of aiming system 100 such as aiming arm 102 and targeting block 110 may be manufactured using various polymeric materials via additive manufacturing, molding, milling, and the like. Such polymeric materials are preferably strong enough to not flex or move such that the aiming holes of aiming system 100 become misaligned with the holes of bone plate 106. The polymers may be reinforced with carbon fiber or other similar fibers. These materials are also temperature form stable such that they can be sterilized when manufacture and then washed for multiple uses. Further, the components of aiming system 100 may be radiolucent or include radiolucent components to increase visibility during an operation. Components such as the handle and bone screws may be manufactured using metallic components via machining, milling, or other similar processes.

Furthermore, although the invention disclosed herein has been described with reference to particular features, it is to be understood that these features are merely illustrative of the principles and applications of the present invention. It is therefore to be understood that numerous modifications, including changes in the sizes of the various features described herein, may be made to the illustrative embodiments and that other arrangements may be devised without departing from the scope of the present invention as defined in the appended claims.

## Claims

1. A targeting guide (100) for aligning a bone plate (106), comprising:
an aiming arm (102);
an attachment portion (138) extending from a proximal end (130) of the aiming arm (102), the attachment portion (138) having an opening (140) removably connectable with a shaft (112) of a handle (104); and
a targeting block (110) removably connectable with the shaft (112), **characterised in that** the targeting block (110) is positioned over the attachment portion (138).

2. The guide (100) of claim 1, wherein the aiming arm (102) extends along a longitudinal axis and has an upper side (116) and an opposing lower side (118), a first sidewall (120) and a second sidewall (122) connecting the upper side (116) to the lower side (118), and a plurality of holes (124) extending through the upper and lower sides (116, 118).

3. The guide (100) of claim 1 or 2, wherein the targeting block (110) has an upper surface (152), an opposing lower surface (154), a sidewall connecting the upper surface (152) to the lower surface (154), and a plurality of apertures (156) extending through the upper and lower surfaces (152, 154).

4. The guide (100) of any one of claims 1 to 3, further comprising an outrigger (132) extending laterally away from the first sidewall (120) of the aiming arm (102), the outrigger (132) including a hole (134) defining an axis extending toward a neck portion (176) of the bone plate (106).

5. The guide (100) of claim 4, wherein the outrigger (132) extends away from the first sidewall and the second sidewall (120, 122) of the aiming arm (102).

6. The guide (100) of claim 4 or 5, wherein the outrigger (132) is integrally attached to the aiming arm (102) or attached to the aiming arm (102) with a fastener.

7. The guide (100) of any one of claims 1 to 6, further comprising a locking collar (108) attachable with the shaft (112) of the handle (104).

8. The guide (100) of any one of claims 1 to 7, wherein the targeting block (110) is substantially pentagonal or substantially circular.

9. The guide (100) of claim 3 or of any one of claims 4 to 8 when depending on claim 3, wherein the sidewall of the targeting block (110) is curved to define a circular targeting block (110).

10. The guide (100) of claim 2 or of any one of claims 3 to 9 when depending on claim 2, wherein each hole of the plurality of holes (124) of the aiming arm (102) aligns with a corresponding plate hole (184, 186) of a shaft portion (174) of the bone plate (106).

11. The guide (100) of claim 3 or of any one of claims 4 to 10 when depending on claim 3, wherein each aperture of the plurality of apertures (156) of the targeting block (110) aligns with a corresponding plate hole (182) of a head portion of the bone plate (106).

12. The guide (100) of any one of claims 1 to 11, wherein the handle (104) is configured to connect the aiming arm (102) to at least one of a head portion (172) and neck portion (176) of the bone plate (106).

13. The guide (100) of any one of claims 1 to 12, wherein the attachment portion (138) is integrally attached to the aiming arm (102).

14. The guide (100) of any one of claims 1 to 13, wherein the shaft (112) of the handle (104) is configured to extend through the opening in the attachment portion (138) and a block opening (157) in the targeting block (110).

15. The guide (100) of any one of claims 1 to 14, wherein the targeting block (110) includes a groove (158) for receiving a portion of the attachment portion (138) and the aiming arm (102).

## Patentansprüche

1. Zielführung (100) zum Ausrichten einer Knochenplatte (106), umfassend:
einen Zielarm (102);
einen Befestigungsabschnitt (138), der sich von einem proximalen Ende (130) des Zielarms (102) erstreckt, wobei der Befestigungsabschnitt (138) eine Öffnung (140) aufweist, die abnehmbar mit einem Schaft (112) eines Handgriffs (104) verbindbar ist; und
einen Zielblock (110), der abnehmbar mit dem Schaft (112) verbindbar ist,
**dadurch gekennzeichnet, dass** der Zielblock (110) über dem Befestigungsabschnitt (138) positioniert ist.

2. Zielführung (100) nach Anspruch 1, wobei sich der Zielarm (102) entlang einer Längsachse erstreckt und eine Oberseite (116) und eine gegenüberliegende Unterseite (118), eine erste Seitenwand (120) und eine zweite Seitenwand (122), welche die Oberseite (116) mit der Unterseite (118) verbinden, und eine Mehrzahl von Löchern (124) aufweist, die sich durch die Ober- und Unterseite (116, 118) erstrecken.

3. Zielführung (100) nach Anspruch 1 oder 2, wobei der Zielblock (110) eine obere Fläche (152), eine gegenüberliegende untere Fläche (154), eine Seitenwand, welche die obere Fläche (152) mit der unteren Fläche (154) verbindet, und eine Mehrzahl von Durchgangsöffnungen (156) aufweist, die sich durch die obere und untere Fläche (152, 154) erstrecken.

4. Zielführung (100) nach einem der Ansprüche 1 bis 3, ferner umfassend einen Ausleger (132), der sich lateral von der ersten Seitenwand (120) des Zielarms (102) weg erstreckt, wobei der Ausleger (132) ein Loch (134) aufweist, das eine Achse definiert, die sich in Richtung eines Halsabschnitts (176) der Knochenplatte (106) erstreckt.

5. Zielführung (100) nach Anspruch 4, wobei sich der Ausleger (132) von der ersten Seitenwand und der zweiten Seitenwand (120, 122) des Zielarms (102) weg erstreckt.

6. Zielführung (100) nach Anspruch 4 oder 5, wobei der Ausleger (132) einstückig am Zielarm (102) angebracht ist oder mit einem Befestigungselement am Zielarm (102) angebracht ist.

7. Zielführung (100) nach einem der Ansprüche 1 bis 6, ferner umfassend einen Verriegelungsbund (108), der am Schaft (112) des Handgriffs (104) anbringbar ist.

8. Zielführung (100) nach einem der Ansprüche 1 bis 7, wobei der Zielblock (110) im Wesentlichen fünfeckig oder im Wesentlichen kreisförmig ist.

9. Zielführung (100) nach Anspruch 3 oder nach einem der Ansprüche 4 bis 8, soweit rückbezogen auf Anspruch 3, wobei die Seitenwand des Zielblocks (110) gekrümmt ist, um einen kreisförmigen Zielblock (110) zu definieren.

10. Zielführung (100) nach Anspruch 2 oder nach einem der Ansprüche 3 bis 9, soweit rückbezogen auf Anspruch 2, wobei jedes Loch der Mehrzahl von Löchern (124) des Zielarms (102) mit einem entsprechenden Plattenloch (184, 186) eines Schaftabschnitts (174) der Knochenplatte (106) ausgerichtet ist.

11. Zielführung (100) nach Anspruch 3 oder nach einem der Ansprüche 4 bis 10, soweit rückbezogen auf Anspruch 3, wobei jede Durchgangsöffnung der Mehrzahl von Durchgangsöffnungen (156) des Zielblocks (110) mit einem entsprechenden Plattenloch (182) eines Kopfabschnitts der Knochenplatte (106) ausgerichtet ist.

12. Zielführung (100) nach einem der Ansprüche 1 bis 11, wobei der Handgriff (104) dazu eingerichtet ist, den Zielarm (102) mit einem Kopfabschnitt (172) und/oder einem Halsabschnitt (176) der Knochenplatte (106) zu verbinden.

13. Zielführung (100) nach einem der Ansprüche 1 bis 12, wobei der Befestigungsabschnitt (138) einstückig am Zielarm (102) angebracht ist.

14. Zielführung (100) nach einem der Ansprüche 1 bis 13, wobei der Schaft (112) des Handgriffs (104) dazu eingerichtet ist, sich durch die Öffnung im Befestigungsabschnitt (138) und eine Blocköffnung (157) im Zielblock (110) zu erstrecken.

15. Zielführung (100) nach einem der Ansprüche 1 bis 14, wobei der Zielblock (110) eine Nut (158) zur Aufnahme eines Abschnitts des Befestigungsabschnitts (138) und des Zielarms (102) umfasst.

## Revendications

1. Guide de ciblage permettant d'aligner une plaque vissée (106),
comprenant:
un bras de visée (102);
une partie de fixation (138) s'étendant à partir d'une extrémité proximale (130) du bras de visée (102), la partie de fixation (138) comportant une ouverture (140) pouvant être reliée de manière amovible à une tige (112) d'une poignée (104); et
un bloc de ciblage (110) pouvant être relié de manière amovible à la tige (112), **caractérisé en ce que** le bloc de ciblage (110) est positionné au-dessus de la partie de fixation (138).

2. Guide (100) selon la revendication 1, dans lequel le bras de visée (102) s'étend le long d'un axe longitudinal et comporte une face supérieure (116) et une face inférieure (118) en regard, une première paroi latérale (120) et une deuxième paroi latérale (122) reliant la face supérieure (116) à la face inférieure (118), et une pluralité de trous (124) s'étendant à travers les faces supérieure et inférieure (116, 118).

3. Guide (100) selon la revendication 1 ou 2, dans lequel le bloc de ciblage (110) comporte une surface supérieure (152), une surface inférieure (154) en regard, une paroi latérale reliant la surface supérieure (152) à la surface inférieure (154), et une pluralité d'ouvertures (156) s'étendant à travers les surfaces supérieure et inférieure (152, 154).

4. Guide (100) selon l'une quelconque des revendications 1 à 3, comprenant en outre un bras porteur (132) s'étendant latéralement à partir de la première paroi latérale (120) du bras de visée (102), le bras porteur (132) comportant un trou (134) délimitant un axe s'étendant vers une partie col (176) de la plaque vissée (106).

5. Guide (100) selon la revendication 4, dans lequel le bras porteur (132) s'étend à partir de la première paroi latérale et de la deuxième paroi latérale (120, 122) du bras de visée (102).

6. Guide (100) selon la revendication 4 ou 5, dans lequel le bras porteur(132) est fixé d'un seul tenant au bras de visée (102) ou est fixé au bras de visée (102) à l'aide d'un élément de fixation.

7. Guide (100) selon l'une quelconque des revendications 1 à 6, comprenant en outre une bague de verrouillage (108) pouvant être fixée à la tige (112) de la poignée (104).

8. Guide (100) selon l'une quelconque des revendications 1 à 7, dans lequel le bloc de ciblage (110) est sensiblement pentagonal ou sensiblement circulaire.

9. Guide (100) selon la revendication 3 ou selon l'une quelconque des revendications 4 à 8, lorsqu'elles dépendent de la revendication 3, dans lequel la paroi latérale du bloc de ciblage (110) est incurvée de manière à former un bloc de ciblage circulaire (110).

10. Guide (100) selon la revendication 2 ou selon l'une quelconque des revendications 3 à 9, lorsqu'elles dépendent de la revendication 2, dans lequel chaque trou de la pluralité de trous (124) du bras de visée (102) s'aligne avec un trou de plaque correspondant (184, 186) d'une partie tige (174) de la plaque vissée (106).

11. Guide (100) selon la revendication 3 ou selon l'une quelconque des revendications 4 à 10, lorsqu'elles dépendent de la revendication 3, dans lequel chaque ouverture de la pluralité d'ouvertures (156) du bloc de ciblage (110) s'aligne avec un trou de plaque correspondant (182) d'une partie de tête de la plaque vissée (106).

12. Guide (100) selon l'une quelconque des revendications 1 à 11, dans lequel la poignée (104) est conçue pour relier le bras de visée (102) à au moins l'une parmi une partie tête (172) ou une partie col (176) de la plaque vissée (106).

13. Guide (100) selon l'une quelconque des revendications 1 à 12, dans lequel la partie de fixation (138) est fixée d'un seul tenant au bras de visée (102).

14. Guide (100) selon l'une quelconque des revendications 1 à 13, dans lequel la tige (112) de la poignée (104) est conçue pour s'étendre à travers l'ouverture pratiquée dans la partie de fixation (138) et une ouverture de bloc (157) pratiquée dans le bloc de ciblage (110).

15. Guide (100) selon l'une quelconque des revendications 1 à 14, dans lequel le bloc de ciblage (110) comprend une rainure (158) destinée à recevoir une partie de la partie de fixation (138) et le bras de visée (102).
